# EUROPEAN PATENT APPLICATION

(11) **EP 1 621 200 A1**
(43) Date of publication of application: **01.02.2006**
(21) Application number: 04291896.1
(22) Date of filing: 26.07.2004
(51) Int. Cl.: A61K 31/60, A61K 31/4365, A61K 31/216, A61P 7/02

(54) **Pharmaceutical combinations containing an inhibitor of platelet aggregation and a fibrate**

(71) Applicant: Fournier Laboratories Ireland Limited, Carrigtwohill, Co. Cork (IE)
(72) Inventor: Edgar, Alan, 21490 Saint Julien (FR); Junien, Jean-Louis, 92310 Sevres (FR); Wilkins, Michael, Middleton, co Cork (IE)
(74) Representative: Hubert, Philippe

(57) **Abstract**

The present invention relates to a novel pharmaceutical combination, containing an inhibitor of platelet aggregation and a fibrate, where the inhibitor of platelet aggregation is preferably either aspirin or clopidogrel.

Such a pharmaceutical combination of an inhibitor of platelet aggregation and a fibrate is expected to be useful in the treatment and/or prevention of myocardial infarction (heart attack), cardiac arrest, peripheral vascular disease (including symptomatic carotid artery disease), congestive heart failure, ischemic heart disease, angina pectoris (including unstable angina), sudden cardiac death, unstable angina, as well as cerebrovascular events such as cerebral infarction, cerebral thrombosis, cerebral ischemia and transient ischemic attack, disorders related to bypass operations (angioplasty), fitting of endovascular prostheses and restenosis, and inflammatory disorders, including arthritic conditions such as rheumatoid arthritis and osteoarthritis, as well as asthma or related airway or respiratory inflammatory disorders.

## Description

The present invention relates to a novel pharmaceutical combination, containing an inhibitor of platelet aggregation and a fibrate. The pharmaceutical combination of an inhibitor of platelet aggregation and a fibrate is applied in the framework of the present invention in particular to reduce the risk of a patient suffering a cardiovascular event, such as myocardial infarction (heart attack) or a stroke.

The inhibitor of platelet aggregation in the present invention is preferably either aspirin or clopidogrel.

### Background of the invention

Acetylsalicylic acid (ASA), more commonly known as aspirin, is known to reduce the risk of cardiovascular events (such as myocardial infarction) or cerebrovascular events (such as strokes) when administered long-term to patients at risk for such events in low daily doses (of the order of 100 mg, rather lower than typical doses taken in order to achieve an analgesic effect). Aspirin is in effect known to act as an irreversible "suicide" inhibitor of the cyclooxygenase enzyme needed along the route from arachidonic acid to prostaglandins and the platelet aggregation-inducing thromboxane A₂ (TxA₂).

Clopidogrel is a specific and irreversible inhibitor of platelet adenylate cyclase-coupled ADP receptors, and prevents the binding of fibrinogen to its corresponding platelet receptors (GP-IIb/IIIa glycoprotein). Clopidogrel is sold under the name PLAVIX® and is used as an anti-thrombotic.

Fibrates, which are PPARα activators, have been documented to lower plasma triglycerides and cholesterol levels and to be beneficial in the prevention of ischemic heart disease in individuals with elevated levels of LDL cholesterol. They can also modestly decrease elevated fibrinogen and PAI-1 levels. Fibrate compounds, e.g., gemfibrozil, fenofibrate, bezafibrate, and ciprofibrate, elevate the level of plasma HDL cholesterol. The ability of fibrates to inhibit platelet aggregation by themselves has been reported in the literature (Renaud et al., Haemostasis, 1979, 8, 82-95).

### Summary of the invention

It has now surprisingly been found that co-administration of an inhibitor of platelet aggregation and a fibrate results in effects that are expected to be beneficial for subjects at risk of developing cardiovascular disease and/or at risk of suffering a cardiovascular event. In particular, it has been found that the platelet aggregation-inhibiting effect of a combination of aspirin and a fibrate is greater than that of the sum of the effects of the two components used separately, i.e. there is synergy between the two components. In particular, at an arachidonic acid concentration of 1 mM, rat platelet aggregation is strongly inhibited when rats are treated by a combination of aspirin and a fibrate, whereas each of the two products of the pharmaceutical combination is inactive or not very active in inhibiting aggregation when used alone at the same doses in the presence of 1 mM arachidonic acid. Such a finding enables the use of smaller doses of the two components to be envisaged, leading to a reduction in the side effects associated with these active substances.

Accordingly, the present invention relates a novel pharmaceutical combination containing an inhibitor of platelet aggregation, a fibrate and one or more pharmaceutically acceptable excipients. The present invention relates further to the use of an inhibitor of platelet aggregation, a fibrate and one or more pharmaceutically acceptable excipients in the manufacture of a medicament for the prevention or treatment of cardiovascular diseases or events, and to a method for the prevention or treatment of cardiovascular diseases or events, comprising co-administering an effective dose of an inhibitor of platelet aggregation and a fibrate. The fibrate used in this method may be selected from the group consisting of gemfibrozil, fenofibrate, bezafibrate, clofibrate and ciprofibrate.

The preferred fibrate for the new pharmaceutical combination of the invention and new use is either fenofibrate or its active metabolite, fenofibric acid.

The preferred inhibitor of platelet aggregation in the framework of the present invention is aspirin or clopidogrel.

The most preferred pharmaceutical combinations of the present invention contain as active ingredients aspirin and fenofibrate, or clopidogrel and fenofibrate.

### Detailed description of the invention

In the framework of the present invention, by "aspirin" it is intended to refer not only to the purified substance of aspirin, but also to salts, solvates and complexes (such as inclusion complexes) of aspirin.

A number of different types of aspirin dosage form are known, including tablets, dispersible/buffered tablets and enteric coated tablets. Any of these forms of aspirin can be envisaged as the basis for an aspirin-fibrate combination within the framework of the present invention.

Concerning the dispersible/buffered tablets of aspirin, these typically contain basic metal oxides, hydroxide or carbonates to neutralize the acidity of aspirin, and thereby alleviate gastrointestinal (GI) bleeding when prescribed to patients long-term. Such agents may include calcium carbonate, magnesium hydroxide, aluminium hydroxide and mixed hydroxides and carbonates based on these species. The amount of such an agent will depend on the amount of aspirin being used, but will lie normally in the range of 10 to 1000 mg per unit dose.

Preferably, whether the aspirin used in the framework of the present invention arises from a tablet-, dispersible/buffered tablet- or enteric coated tablet-type formulation, it will be prepared in the presence of the excipients including one or more of the group consisting of: citric acid, calcium carbonate, saccharin sodium, diethylphthalate, cellulose acetate phthalate, gelatine, calcium sulphate, sodium benzoate, titanium dioxide and acacia powder.

Clopidogrel is an inhibitor of platelet aggregation of the thienopyridine class, and has the chemical name methyl (4)-(S)-α-(o-chlorophenyl)-6,7-dihydrothieno[3,2-c]pyridine-5-acetate.

In the framework of the present invention, by "clopidogrel" it is intended to refer not only to the clopidogrel as an acetate ester, but also to the free carboxylic acid form of clopidogrel, other esters of this free acid, and/or pharmaceutically acceptable salts thereof. In particular, addition salts of clopidogrel with sulphuric acid such as those formed with sulphuric acid (clopidogrel sulphate and clopidogrel hydrogen sulphate salts) are intended to be covered by the term "clopidogrel".

In the present invention, fibrates are defined as PPARα agonists (peroxisome proliferator activated receptor alpha agonists), including fibric acid derivatives and pharmaceutically acceptable salts and esters of such fibric acid derivatives. Fibric acid derivatives lower the levels of triglyceride-rich lipoproteins, such as VLDL, raise HDL levels, and have variable effects on LDL levels. The effects on VLDL levels appear to result primarily from an increase in lipoprotein lipase activity, especially in muscle. This leads to enhanced hydrolysis of VLDL triglyceride content and an enhanced VLDL catabolism. Fibric acid agents also may alter the composition of the VLDL, for example, by decreasing hepatic production of apoC-III, an inhibitor of lipoprotein lipase activity. These compounds are also reported to decrease hepatic VLDL triglyceride synthesis, possibly by inhibiting fatty acid synthesis and by promoting fatty acid oxidation.

Fibrate compounds include, but are not limited to, gemfibrozil, fenofibrate, bezafibrate, clofibrate, ciprofibrate, and analogs, derivatives and pharmaceutically acceptable salts thereof.

Fenofibrate is commercially available as Tricor® capsules. Each capsule contains 67 mg of micronized fenofibrate.

Fenofibric acid, the active metabolite of fenofibrate, lowers plasma triglycerides apparently by inhibiting triglyceride synthesis, resulting in a reduction of VLDL released into the circulation, and also by stimulating the catabolism of triglyceride rich lipoproteins (i.e. VLDL).

Clofibrate is commercially available as Atromid-S® capsules. Each capsule contains 500 mg of clofibrate. Clofibrate lowers elevated serum lipids by reducing the very low-density lipoprotein fraction rich in triglycerides. Serum cholesterol may be decreased. It may inhibit the hepatic release of lipoproteins (particularly VLDL) and potentiate the action of lipoprotein lipase. The recommended daily dose of clofibrate is 2 g, administered in divided doses.

Gemfibrozil is commercially available as Lopid® tablets. Each tablet contains 600 mg of gemfibrozil. Gemfibrozil is a lipid regulating agent that decreases serum triglycerides and very low density lipoprotein cholesterol, and increases high density lipoprotein cholesterol. The recommended daily dose of gemfibrozil is 1200 mg, administered in two divided doses.

Fibrates include PPARα agonists; the PPARα agonists may be identified according to an assay described in US Patent 6,008,239. Pharmaceutically acceptable salts and esters of PPARα agonists are likewise included within the scope of this invention. Compounds which are PPARα agonists include compounds such as those described in US Patent 6,008,239, WO 97/27847, WO 97/27857, WO 97/28115, WO 97/28137 and WO 97/28149. Fibrate compounds as described in WO 92/10468 and WO 01/80852 are also incorporated by reference herein.

According to the present invention, the preferred fibrate is fenofibrate, also known chemically as 2-[4-(4-chlorobenzoyl)phenoxy]-2-methyl-propanoic acid, 1-methylethyl ester. Also preferred in the present invention as active fibrate substance is the free acid fenofibric acid, the active metabolite of fenofibrate, the chemical name of the free acid being 2-[4-(4-chlorobenzoyl)phenoxy]-2-methyl-propanoic acid.

The fibrate can be of a reduced particle size. The fibrate can for example be micronised or co-micronised with a surfactant. Any surfactant is suitable, whether it be amphoteric, non-ionic, cationic or anionic. Examples of such surfactants are: sodium lauryl sulfate, monooleate, monolaurate, monopalmitate, monostearate or another ester of polyoxyethylene sorbitane, sodium dioctylsulfosuccinate (DOSS; also known as sodium docusate), lecithin, stearylic alcohol, cetostearylic alcohol, cholesterol, polyoxyethylene ricin oil, polyoxyethylene fatty acid glycerides, a poloxamer and mixtures thereof. The preferred surfactant is sodium lauryl sulfate, which can be (co-)micronized with fenofibrate such as described in EP 0 330 532.

In one preferred embodiment of the invention, the fibrate particles have an average particle size of less than about 20 µm, preferably of less than about 10 µm.

The fibrate can also be in the form of nanoparticles which can be obtained using, for example, milling, homogenization, or precipitation techniques. Methods of making nanoparticulate compositions are described in U.S. Patent Nos. 5,518,187, 5,718,388, 5,862,999, 5,665,331, 5,662,883, 5,560,932, 5,543,133, 5,534,270, 5,510,118, 5,470,583, and US patent application 2003/0 224 058, all of which are specifically incorporated by reference.

Nanoparticulate fibrate dispersions can be obtained by milling a fibrate, preferably fenofibrate, and by dispersing the fibrate particles in a liquid dispersion medium in which the fibrate is poorly soluble, followed by applying mechanical means in the presence of grinding media to reduce the particle size of the fibrate to the desired effective average particle size. The dispersion medium can be, for example, water, sunflower oil, ethanol, t-butanol, glycerin, polyethylene glycol (PEG), hexane, or glycol. A preferred dispersion medium is water.

The fibrate, preferably fenofibrate, particles can be reduced in size in the presence of at least one surface stabilizer. Alternatively, the fibrate particles can be contacted with one or more surface stabilizers after attrition. Other compounds, such as a diluent, can be added to the fibrate/surface stabilizer composition during the size reduction process. Dispersions can be manufactured continuously or in a batch mode. Combinations of more than one surface stabilizer can be used. Useful surface stabilizers which can be employed include, but are not limited to, known organic and inorganic pharmaceutical excipients. Such excipients include various polymers, low molecular weight oligomers, natural products, and surfactants. Surface stabilizers include nonionic, anionic, cationic, ionic, and zwitterionic surfactants. Suitable surface stabilizers are those mentioned in US patent application 2003/0224058, the contents of which are incorporated herein by reference, and described below.

Another method of forming the desired nanoparticulate fibrate, preferably fenofibrate, is by microprecipitation. This is a method of preparing stable dispersions of poorly soluble active agents in the presence of one or more surface stabilisers and one or more colloid stability enhancing surface active agents free of any trace of toxic solvents or of solubilized heavy metal impurities. Such a method comprises, for example: (1) dissolving the fibrate in a suitable solvent; (2) adding the formulation from step (1) to a solution comprising at least one surface stabilizer; and (3) precipitating the formulation from step (2) using an appropriate non-solvent. The method can be followed by removal of any formed salt, if present, by dialysis or diafiltration and concentration of the dispersion by conventional means.

Nanoparticulate fibrate can also be obtained by homogenization: exemplary homogenisation methods of preparing active agent nanoparticulate compositions are described in U.S. Patent No. 5,510,118. Such a method comprises dispersing particles of a fibrate, preferably fenofibrate, in a liquid dispersion medium, followed by subjecting the dispersion to homogenisation to reduce the particle size of the fibrate to the desired particle size. The fibrate particles can be reduced in size in the presence of at least one surface stabilizer. Alternatively, the fibrate particles can be contacted with one or more surface stabilizers either before or after attrition. Other compounds, such as a diluent, can be added to the fenofibrate/surface stabilizer composition either before, during, or after the size reduction process. Dispersions can be manufactured continuously or in a batch mode.

According to the invention, the nanoparticulate fibrate has an average particle size of less than about 2000 nm, preferably less than about 1000 nm, more preferably less than about 100 nm, most preferably less than about 50 nm.

As used in this application, "co-administration" means the administration of two or more compounds to the same patient, within a time period of up to about three to about four hours. For example, co-administration encompasses (1) simultaneous administration of a first and second compound; (2) administration of a first compound, followed by administration of a second compound about 2 hours after administration of the first compound; and (3) administration of a first compound, followed by administration of a second compound about 4 hours after administration of the first compound. As described herein, the present invention encompasses co-administration of aspirin and a fibrate, or clopidogrel and a fibrate, to a patient.

In one preferred embodiment of the present invention, a kit is provided containing a plurality of dosage units, some of which contain a fibrate only, and some of which contain aspirin only (or clopidogrel only). The kit will contain written instructions for the patient concerning the separate administration of both active substances, i.e. aspirin (or clopidogrel) on the one hand, and a fibrate on the other hand.

In a preferred and advantageous embodiment of the present invention, single dosage units containing both active substances, aspirin (or clopidogrel) and a fibrate, are provided, combined in a single pharmaceutical formulation. By this means, simultaneous administration of both active substances to a patient is achieved.

The compositions of the invention are preferably administered enterally or parenterally (parenteral administration includes subcutaneous, intramuscular, intradermal, intramammary, intravenous, and other administrative methods known in the art). The subject combinations can also be administered by infusion techniques, in the form of sterile injectable aqueous or olagenous suspensions, formulated according to the known art using those suitable dispersing of wetting agents and suspending agents, or other acceptable agents. The subject combination can also be administered by inhalation, in the form of aerosols or solutions for nebulizers, or rectally in the form of suppositories prepared by mixing the drug with a suitable non irritating excipient, which is solid at ordinary temperature but liquid at the rectal temperature and will therefore melt in the rectum to release the drug. Such materials are cocoa butter and polyethylene glycols.

In a preferred embodiment, the compositions of the present invention are in a form suitable for oral administration, and more preferably in a solid form such as tablets, granules, capsules or powders.

A preferred embodiment of the present invention comprises a pharmaceutical composition, comprising a therapeutically-effective amount of a combination of an inhibitor of platelet aggregation and a fibrate in association with at least one pharmaceutically-acceptable carrier, adjuvant, or other excipient, it being understood that the carrier, adjuvant, or other excipient does not have a direct pharmacological effect as an active substance in the framework of the invention, although the carrier, adjuvant or other excipient will naturally influence the rate at which the active substances of the pharmaceutical combinations of the invention are released into the circulatory system of the patient. Although the presence of a third or subsequent active substance, beyond the inhibitor of platelet aggregation and the fibrate, is not excluded in the present invention, compositions according to the present invention will preferably contain only the inhibitor of platelet aggregation and the fibrate as the two sole active substances. The preferred compositions according to the present invention will therefore consist essentially of a single inhibitor of platelet aggregation (aspirin or clopidogrel) combined with a single fibrate, the other elements present in the composition being excipients not having intrinsic pharmacological activity and therefore not materially modifying the nature of the actions of the inhibitor of platelet aggregation + fibrate combination in the functioning of the present invention.

As discussed above, the preferred fibrate for the new pharmaceutical combination of the invention and is either fenofibrate or its active metabolite, fenofibric acid, and the preferred inhibitor of platelet aggregation in the framework of the present invention is aspirin or clopidogrel. Consequently, the most preferred pharmaceutical combinations of the present invention thus contain as sole active ingredients (i) aspirin and fenofibrate, or (ii) clopidogrel and fenofibrate.

For preparing pharmaceutical compositions from the compounds of this invention, inert, pharmaceutically acceptable carriers (excipients) can be either solid or liquid. Solid form preparations include powders, tablets, coated tablets, dragees, troches, lozenges, dispersible granules, capsules, and sachets.

Pharmaceutical compositions according to the present invention may comprise one or more excipients known in the art. Such excipients include (a) surface stabilizers, (b) binders, (c) filling agents, (d) lubricating agents, (e) glidants, (f) suspending agents, (g) sweeteners, (h) flavoring agents, (i) preservatives, (j) buffers, (k) wetting agents, (I) disintegrants, (m) effervescent agents, (n) humectants, (o) solution retarders, (p) absorption accelerators, (q) adsorbents.

### a) Surface stabilizers

Examples of surface stabilizers which may be used within the framework of of the invention are polymers, low molecular weight oligomers, natural products, and surfactants, including nonionic, anionic, cationic, ionic, and zwitterionic surfactants, as well as mixtures thereof.

Representative examples of surface stabilizers include hydroxypropyl methylcellulose (now known as hypromellose), hydroxypropylcellulose, polyvinylpyrrolidone, sodium lauryl sulfate, sodium dioctylsulfosuccinate (also known as sodium docusate), gelatin, casein, lecithin (phosphatides), dextran, gum acacia, cholesterol, tragacanth, stearic acid, benzalkonium chloride, calcium stearate, glycerol monostearate, cetostearyl alcohol, cetomacrogol emulsifying wax, sorbitan esters (e.g. the commercially available Spans® such as Span® 80 and Span® 20), polyoxyethylene alkyl ethers (e.g., macrogol ethers such as cetomacrogol 1000), polyoxyethylene castor oil derivatives (polyoxols) (e.g. the commercially available Cremophors®), polyoxyethylene sorbitan fatty acid esters (e.g., the commercially available Tweens® such as e.g., Tween 20® and Tween 80® (ICI Speciality Chemicals)); polyethylene glycols (e.g., Carbowaxs 3550® and 934® (Union Carbide)), polyoxyethylene stearates, colloidal silicon dioxide, phosphates, carboxymethylcellulose calcium, carboxymethylcellulose sodium, methylcellulose, hydroxyethylcellulose, hypromellose phthalate, noncrystalline cellulose, magnesium aluminium silicate, triethanolamine, polyvinyl alcohol (PVA), 4-(1,1,3,3-tetramethylbutyl)-phenol polymer with ethylene oxide and formaldehyde (also known as Tyloxapol®, Superione®, and Triton@), poloxamers (e.g., Pluronics F68® and F108®, which are block copolymers of ethylene oxide and propylene oxide); poloxamines (e.g., Tetronic 908®, also known as Poloxamine 908®, which is a tetrafunctional block copolymer derived from sequential addition of propylene oxide and ethylene oxide to ethylenediamine (BASF Wyandotte Corporation, Parsippany, N.J.)); Tetronic 1508® (T-1508) (BASF Wyandotte Corporation); Triton X-200®, which is an alkyl aryl polyether sulfonate (Rohm and Haas); Crodestas F-110®, which is a mixture of sucrose stearate and sucrose distearate (Croda Inc.); p-isononylphenoxypoly-(glycidol), also known as Olin-10G® or Surfactant 10-G® (Olin Chemicals, Stamford, Conn.); Crodestas SL-40® (Croda, Inc.); and SA90HC0® (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl β-D-glucopyranoside; n-heptyl β-D-thioglucoside; n-hexyl β-D-glucopyranoside; nonanoyl-N-methylglucamide; n-nonyl β-D-glucopyranoside; octanoyl-N-methylglucamide; n-octyl- β-D-glucopyranoside; octyl β-D-thioglucopyranoside; PEG-phospholipid, PEG-cholesterol, PEG-cholesterol derivative, PEG-vitamin A, PEG-vitamin E, lysozyme, random copolymers of vinyl pyrrolidone and vinyl acetate, and the like.

If desirable, nanoparticulate fibrate, preferably fenofibrate, compositions of the invention can be formulated to be phospholipid-free.

Examples of useful cationic surface stabilizers include, but are not limited to, polymers, biopolymers, polysaccharides, cellulosics, alginates, phospholipids, and nonpolymeric compounds, such as zwitterionic stabilizers, poly-n-methylpyridinium, anthryl pyridinium chloride, cationic phospholipids, chitosan, polylysine, polyvinyl imidazole, polybrene, polymethylmethacrylate trimethylammoniumbromide bromide (PMMTMABr), hexyldesyltrimethylammonium bromide (HDMAB), and polyvinylpyrrolidone-2-dimethylaminoethyl methacrylate dimethyl sulfate.

Other useful cationic stabilizers include, but are not limited to, cationic lipids, sulfonium, phosphonium, and quarternary ammonium compounds, such as stearyltrimethylammonium chloride, benzyl-di(2-chloroethyl)ethylammonium bromide, coconut trimethyl ammonium chloride or bromide, coconut methyl dihydroxyethyl ammonium chloride or bromide, decyl triethyl ammonium chloride, decyl dimethyl hydroxyethyl ammonium chloride or bromide, alkyl-dimethyl hydroxyethyl ammonium chloride or bromide, coconut dimethyl hydroxyethyl ammonium chloride or bromide, myristyl trimethyl ammonium methyl sulphate, lauryl dimethyl benzyl ammonium chloride or bromide, lauryl dimethyl (ethenoxy)₄ ammonium chloride or bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl (C₁₂₋₁₄) dimethyl 1-napthylmethyl ammonium chloride, trimethylammonium halide, alkyl-trimethylammonium salts and dialkyl-dimethylammonium salts, lauryl trimethyl ammonium chloride, ethoxylated alkyamidoalkyldialkylammonium salt and/or an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride and dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride (ALIQUAT® 336), POLYQUAT® 10, tetrabutylammonium bromide, benzyl trimethylammonium bromide, choline esters (such as choline esters of fatty acids), benzalkonium chloride, stearalkonium chloride compounds (such as stearyltrimonium chloride and distearyldimonium chloride), cetyl pyridinium bromide or chloride, halide salts of quaternized polyoxyethylalkylamines, MIRAPOL® and ALKAQUAT® (Alkaril Chemical Company), alkyl pyridinium salts; amines, such as alkylamines, dialkylamines, alkanolamines, polyethylenepolyamines, N,N-dialkylaminoalkyl acrylates, and vinyl pyridine, amine salts, such as lauryl amine acetate, stearyl amine acetate, alkylpyridinium salt, and alkylimidazolium salt, and amine oxides; imide azolinium salts; protonated quaternary acrylamides; methylated quaternary polymers, such as poly[diallyl dimethylammonium chloride] and poly-[N-methyl vinyl pyridinium chloride]; and cationic guar.

Such exemplary cationic surface stabilizers and other useful cationic surface stabilizers are described in J. Cross and E. Singer, Cationic Surfactants: Analytical and Biological Evaluation (Marcel Dekker, 1994); P. and D. Rubingh (Editor), Cationic Surfactants: Physical Chemistry (Marcel Dekker, 1991); and J. Richmond, Cationic Surfactants: Organic Chemistry, (Marcel Dekker, 1990).

Preferred surface stabilizers for combinations of a fibrate and an inhibitor of platelet aggregation according to the present invention are sodium lauryl sulphate and/or sodium dioctylsulfosuccinate (also known as sodium docusate).

### b) Binders

Examples of binders which may be used within the framework of the present invention include acacia gum, alginic acid, carboxymethylcellulose, carboxymethylcellulose sodium, carboxyethylcellulose, dextrin, ethylcellulose, gelatin, guar gum, hydrogenated vegetable oil, hydroxyethylcellulose, hydroxypropylcellulose, hydroxypropylmethylcellulose, liquid glucose, magnesium aluminium silicate, maltodextrin, methylcellulose, polymethacrylates, povidone (polyvinylpyrrolidone), pre-gelatinised starch, sodium alginate, starch, xanthan gum, tragacanth gum, zein and mixtures thereof.

Preferred binders for combinations of a fibrate and an inhibitor of platelet aggregation according to the present invention are: hydroxypropylcellulose, hydroxypropylmethylcellulose and/or povidone (polyvinylpyrrolidone).

### c) Filling agents / diluents

Examples of filling agents / diluents which may be used within the framework of the present invention include calcium carbonate, calcium sulphate, sucrose, dextrates, dextrin, calcium phosphate, glyceryl palmitostearate, hydrogenated vegetable oil, kaolin, lactose, magnesium carbonate, magnesium oxide, maltodextrin, mannitol, microcrystalline cellulose, silicified microcrystalline cellulose, polymethacrylates, potassium chloride, powdered cellulose, pre-gelatinised starch, sodium chloride, sorbitol, starch, talc and calcium phosphate and mixtures thereof.

Preferred filling agents / diluents for combinations of a fibrate and an inhibitor of platelet aggregation according to the present invention are: sucrose, lactose, mannitol and/or starch.

### d) Lubricating agents

Examples of lubricating agents (lubricants) which may be used within the framework of the present invention include calcium stearate, glyceryl monostearate glyceryl palmitostearate, hydrogenated castor oil, hydrogenated vegetable oil, light mineral oil, magnesium stearate, mineral oil, polyethylene glycol, sodium benzoate, sodium lauryl sulphate, sodium stearyl fumarate, stearic acid, talc, magnesium stearate and zinc stearate and mixtures thereof.

Preferred lubricating agents (lubricants) for combinations of a fibrate and an inhibitor of platelet aggregation according to the present invention are: talc and/or magnesium stearate.

### e) Glidants

Examples of glidants which may be used within the framework of the present invention include colloidal silicon dioxide, magnesium trisilicate, powdered cellulose, starch, talc and calcium phosphate and mixtures thereof.

### f) Suspending agents

Examples of suspending agents which may be used within the framework of the present invention include acacia, agar, carageenan, guar gum, sodium alginate, starch, tragacanth, xanthan gum, carmellose sodium, hydroxyethylcellulose, hydroxypropylcellulose, hydroxypropylmethylcellulose, methylcellulose, microcrystalline cellulose, dispersible cellulose, propylene glycol alginate, aluminum magnesium silicate, bentonite, carbomers, colloidal anhydrous silica, polyvinyl alcohol, povidone, and gelatin.

### g) Sweeteners

Examples of sweeteners which may be used within the framework of the present invention include any natural or artificial sweetener, such as sucrose, dextrose, glycerin, lactose, liquid glucose, mannitol, sorbitol, xylitol, acesulfame potassium, aspartame, saccharin, saccharin sodium, sodium cyclamate and mixtures thereof.

### h) Flavoring agents

Examples of flavoring agents which may be used within the framework of the present invention include ethyl maltol, ethyl vanillin, fumaric acid, malic acid, maltol, menthol, vanillin, Magnasweet® (trademark of MAFCO), bubble gum flavor, and fruit flavors, and the like.

### i) Preservatives

Examples of preservatives which may be used within the framework of the present invention include alcohol, benzalkonium chloride, benzethonium chloride, benzoic acid, benzyl alcohol, bronopol, butyl-paraben, cetrimide, chlorhexidine, chlorobutanol, chlorocresol, cresol, ethylparaben, glycerin, imidurea, methylparaben, phenol, phenoxyethanol, phenylethyl alcohol, phenyl mercuric acetate, phenyl mercuric borate, phenylmercuric nitrate, potassium sorbate, propylene glycol, propyl-paraben, sodium benzoate, sodium propionate, sorbic acid and thiomersal.

### (j) Buffers

Examples of buffers which may be used within the framework of the present invention include phosphate, bicarbonate, tris-hydroxymethylethylamine, glycine, borate, citrate.

### (k) Wetting agents

Examples of wetting agents which may be used within the framework of the present invention include cetyl alcohol, sodium lauryl sulphate, poloxamers, polyoxethylene sorbitan fatty acid derivatives, glycerol monostearate.

### (I) Disintegrants

Examples of disintegrants which may be used within the framework of the present invention include alginic acid, carboxymethylcellulose calcium, carboxymethylcellulose sodium, colloidal silicon dioxide, the cross-linked polymer species known as croscarmellose sodium, crospovidone (cross-linked polyvinylpyrrolidone), guar gum, magnesium aluminium silicate, methylcellulose, microcrystalline cellulose, polacrilin sodium, powdered cellulose, pre-gelatinised starch, sodium alginate, sodium starch glycolate, starch, and mixtures thereof.

Preferred disintegrants for combinations of a fibrate and an inhibitor of platelet aggregation according to the present invention are: croscarmellose sodium, crospovidone and/or sodium starch glycolate.

### (m) Effervescent agents

Examples of effervescent agents which may be used within the framework of the present invention include effervescent couples such as an organic acid and a carbonate or bicarbonate. Suitable organic acids include, for example, citric, tartaric, malic, fumaric, adipic, succinic, and alginic acids and anhydrides and acid salts. Suitable carbonates and bicarbonates include, for example, sodium carbonate, sodium bicarbonate, potassium carbonate, potassium bicarbonate, magnesium carbonate, sodium glycine carbonate, L-lysine carbonate, and arginine carbonate. Alternatively, only the sodium bicarbonate component of the effervescent couple may be present.

### (n) Humectants

As an example of humectant which can be used within the framework of the present invention, glycerol may be mentioned.

### (o) Solution retarders

As an example of solution retarder which can be used within the framework of the present invention, paraffin may be mentioned.

### (p) Absorption accelerators

As examples of absorption accelerators which can be used within the framework of the present invention, quaternary ammonium compounds may be mentioned.

### (q) Adsorbents

As examples of adsorbents which can be used within the framework of the present invention, kaolin and bentonite may be mentioned.

Among preferred excipients for a combination of a fibrate and an inhibitor of platelet aggregation according to the present invention, and in particular for a combination of fenofibrate and aspirin, include: sodium lauryl sulphate and/or sodium dioctylsulfosuccinate (also known as sodium docusate) as surface stabilizers; hydroxypropylcellulose, hydroxypropylmethylcellulose and/or povidone (polyvinylpyrrolidone) as binders; sucrose, lactose, mannitol and/or starch as filling agents / diluents (and, in the case of starch, as a compression aid); talc and/or magnesium stearate as lubricating agents (lubricants); and croscarmellose sodium, crospovidone and/or sodium starch glycolate as disintegrants.

In one preferred method of manufacturing a pharmaceutical combination of fenofibrate and aspirin according to the present invention, a combination of fenofibrate and sodium lauryl sulphate, on the one hand, is combined with an aspirin formulation containing excipients that may include one or more of citric acid, calcium carbonate, saccharin sodium, diethylphthalate, cellulose acetate phthalate, gelatine, calcium sulphate, sodium benzoate, titanium dioxide and acacia powder. Subsequently, it is possible to use a wet granulation, spray-drying or direct compression manufacturing processes to prepare a fenofibrate-aspirin combination according to the present invention.

In another preferred method of manufacturing a pharmaceutical combination of fenofibrate and aspirin according to the present invention, a combination of fenofibrate, sodium docusate, hydroxypropylmethylcellulose, sodium lauryl sulphate and sucrose, on the one hand, is combined with an aspirin formulation containing excipients that may include one or more of citric acid, calcium carbonate, saccharin sodium, diethylphthalate, cellulose acetate phthalate, gelatine, calcium sulphate, sodium benzoate, titanium dioxide and acacia powder. Subsequently, it is possible to use a wet granulation or spray-drying or manufacturing processes to prepare a fenofibrate-aspirin combination according to the present invention.

Liquid pharmaceutical formulations according to the present invention include preparations for parenteral injection as well as formulations for oral administration.

For parenteral injection, liquid preparations can be formulated in solution such as in polyethylene glycol solution.

Liquid dosage forms for oral administration include pharmaceutically acceptable emulsions, solutions, suspensions, syrups and elixirs. For oral administration, aqueous solutions suitable for oral use can be prepared by dissolving the active component in water and adding suitable colorants, flavours, stabilizing and thickening agents as desired. Ethanol, propylene glycol and other pharmaceutically acceptable non-aqueous solvents may be added to improve the solubility of the active compounds. Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavours, stabilizers, buffers, artificial and natural sweeteners, dispersants, thickeners and solubilizing agents.

As used herein, an "effective amount" means the dose or effective amount to be administered to a patient. The dose or effective amount to be administered to a patient and the frequency of administration to the subject can be readily determined by one of ordinary skill in the art by the use of known techniques and by observing results obtained under analogous circumstances. In determining the effective amount or dose, a number of factors are considered by the attending diagnostician, including but not limited to, the potency and duration of action of the compounds used; the nature and severity of the illness to be treated as well as on the sex, age weight, general health and individual responsiveness of the patient to be treated, and other relevant circumstances.

The compositions of the invention contain therapeutically effective amounts of the various active substances. The expression "therapeutically-effective" indicates the capability of an agent to prevent, or reduce the severity of, the disorder, while avoiding adverse side effects typically associated with alternative therapies. The expression "therapeutically-effective" is to be understood to be equivalent to the expression "effective for the treatment, prevention, or inhibition", and both are intended to qualify the amount of each agent for use in the combination therapy, which will achieve the goal of improvement in the prevention or treatment of cardiovascular diseases and events and related conditions.

Compositions according to the present invention, and in particular single dosage units containing both active substances (aspirin and a fibrate) may preferably be presented as "once-a-day" formulations, where the amounts of active substances present are such that a single administration per day provides an appropriate therapeutic effect. It is however not excluded that "twice-a-day", "thrice-a-day" or "four-times-a-day" formulations could be used, requiring two, three of four administrations per day, respectively, in order to provide the patient with therapeutically effective amounts of the active substances.

Concerning aspirin, an effective daily dose in the framework of the present invention, e.g. for preventing or treating of cardiovascular diseases and/or events, lies in the region of about 5 to about 1500 mg/day, preferably from about 10 to about 650 mg/day, more preferably from about 20 to about 400 mg/day, even more preferably from about 50 to about 325 mg/day. More preferable still is a dose of about 75 to about 160 mg/day. Two most favoured aspirin doses are about 75 mg/day and about 81 mg/day.

Concerning clopidogrel, an effective daily dose in the framework of the present invention, e.g. for preventing or treating of cardiovascular diseases and/or events, lies in the region of about 10 to about 1000 mg/day, preferably from about 25 to about 600 mg/day, more preferably from about 50 to about 100 mg/day.

Concerning the fibrate, an effective daily dose is in the range of about 10 to about 3000 mg/day (given in one or more doses), preferably about 10 to about 300 mg/day and most preferably about 40 to about 300 mg/day. A once-a-day formulation containing a fibrate according to the present invention, such as the preferred fenofibrate, will thus contain from about 40 to about 300 mg of fibrate active ingredient.

In the case of an aspirin-fibrate combination, the weight ratio of administered aspirin to administered fibrate will thus lie in the range from about 1:600 to about 150:1 (aspirin:fibrate), preferably from about 1:30 to about 40:1, and more preferably from about 1:15 to about 15:1. In the case of a single dosage unit combining both active substances, such a weight ratio corresponds to the weight ratio of the two active substances incorporated in that single dosage unit (such as a tablet for oral administration).

In the case of a clopidogrel-fibrate combination, the weight ratio of administered clopidogrel to administered fibrate will thus lie in the range from about 1:300 to about 100:1 (clopidogrel:fibrate), preferably from about 1:12 to about 60:1, and more preferably from about 1:6 to about 2.5:1.

As mentioned above, the drug combination of the present invention is expected to be useful in the treatment and/or prevention of "cardiovascular events and diseases", a group which as used herein is intended to include myocardial infarction (heart attack), cardiac arrest, peripheral vascular disease (including symptomatic carotid artery disease), congestive heart failure, ischemic heart disease, angina pectoris (including unstable angina), sudden cardiac death, unstable angina, as well as cerebrovascular events such as cerebral infarction, cerebral thrombosis, cerebral ischemia and transient ischemic attack. Other conditions whose treatment or prevention is aimed at by the compositions of the present invention are: disorders related to bypass operations (angioplasty), fitting of endovascular prostheses and restenosis. It is also envisaged that drug combinations of the present invention will be useful in the treatment of inflammatory disorders, including arthritic conditions such as rheumatoid arthritis and osteoarthritis, and asthma or related airway or respiratory inflammatory disorders.

The following examples describe an embodiment of the invention. Other embodiments within the scope of the claims herein will be apparent to one skilled in the art from consideration of the specification or practice of the invention as disclosed herein. It is intended that the specification, together with the examples, be considered to be exemplary only, with the scope and spirit of the invention being indicated by the claims, which follow the examples.

### Examples

A study was carried out to assess the effect of a combination of a fibrate (fenofibrate) and aspirin on platelet aggregation. In this study, fenofibrate and aspirin were co-administered to rats by the oral route *(p.o.)* and the effects on arachidonic acid-induced platelet aggregation were observed.

### Method

40 male OFA-SD (IOPS Caw) rats (C.River, France), weighing in the target range of 300 to 350 g, were included in the study.

They were housed in a temperature (19.5-24.5°C) and relative humidity (45-65%) controlled room with a 12-h light/dark cycle, with *ad libitum* access to filtered tap-water and standard pelleted laboratory chow (SAFE, France) throughout the study. Upon receipt at animal facilities, they were housed 4 per cage and at least a 5-day acclimatization period was observed. Animals were individually identified on the tail.

The animals of this study were split into 5 groups of 8 animals as follows:
Group 1 : vehicle only
Group 2 : Fenofibrate, 300 mg/kg, *p.o*. × 10 days
Group 3 : Aspirin 3 mg/kg, *p.o*. × 1 day
Group 4 : Fenofibrate, 300 mg/kg, *p.o* × 10 days + Aspirin 3 mg/kg, *p.o.* × 1 day
Group 5 : Aspirin 30 mg/kg, *p.o.* × 1 day

At the end of the acclimatization period, rats were identified on the tail and divided into 10 boxes of 4 rats.

They were then weighed and treated orally once a day during ten consecutive days with the vehicle (1% methylcellulose aqueous solution) or the test substance fenofibrate. On the last day of treatment, the animals were also treated with aspirin as described above. Aspirin (acetyl saliclylic acid) was obtained from Sigma, France and fenofibrate was supplied by Laboratoires Fournier, France. Test substances and the vehicle were administered by *p.o* route once a day, in a volume of 5 ml/kg adjusted to the animal weight measured every day before administration.

On the last day of treatment (day 10), 2 hours after the oral gavage, rats were anaesthetised with isoflurane, and blood samples were collected from the abdominal aorta into plastic tubes containing heparin.

PRP (platelet-rich plasma) was then produced by centrifuging the blood obtained at 250 g for 10 minutes using a GPKR centrifuge (Beckman, France). Then samples were then centrifuged again at 2000 g for 10 minutes to obtain the platelet-poor plasma (PPP).

After a platelet count, each PRP sample was adjusted to 6x10⁸ platelets /mL with the needed volume of homologous PPP (using T540 Cell Counter, Beckman, France).

Platelet aggregation was recorded using Born's method (Born, Nature, 1962, 194, 927-929) with 0.3mL of the platelet suspension in disposable glass cuvettes placed in the turbidometric aggregometer (Chrono-Dual 440 aggregometer, Beckman Coulter, France) and stirred at 1100 rev.min⁻¹ at 37°C.

Platelet aggregation was induced by Arachidonic acid [AA] for each PRP sample tested.

Results were reported as the aggregation amplitude measured in mm and expressed as percentage of maximal aggregation.

Statistical analysis consisted in a one-way analysis of variance followed by multiple comparisons versus the vehicle group (Dunnett's test) on % aggregation values. Where the equal variance test failed, a Kruskall-Wallis one-way analysis of variance on ranks was proposed. A difference is considered significant for p<0.05.

### Results

Table 1 below shows the effects of aspirin alone at different doses, fenofibrate alone, and a combination of aspirin and fenofibrate on rat platelet aggregation.

**Table 1: Ex-vivo effects on rat platelet aggregation**

| Treatment group | % aggregation upon addition of 1 mM arachidonic acid |
|---|---|
| Control | 94.1 |
| (n = 7) | (± 3.6) |
| Fenofibrate 300 mg/kg | 94.2 |
| (n = 8) | (± 10.5) |
| Aspirin 3 mg/kg | 85.0 |
| (n = 8) | (± 8.6) |
| Fenofibrate 300 mg/kg + Aspirin 3 mg/kg | 51.9 |
| (n = 6) | (± 13.4) |
| | * |
| Aspirin 30 mg/kg | 0.4 |
| (n = 8) | (± 0.4) |
| | * |

| | |
|---|---|
| N.B. Values are expressed as mean ± SEM. * indicates p<0.05 as compared to Control group. | |

The combination of fenofibrate with aspirin at 3 mg/kg gave rise to reduced aggregation at an arachidonic acid (AA) concentration of 1 mM as compared to the two individual active substances at the same respective concentrations. At this concentration of 1 mM, aspirin alone at 3 mg/kg or fenofibrate alone gave rise to no reduction in platelet aggregation, whereas the combination treatment gives a result showing a statistically significant difference (45% reduction in aggregation) as compared to platelets from untreated control animals. The synergistic effect of fenofibrate and aspirin in inhibiting platelet aggregation is thus demonstrated for an arachidonic acid (AA) concentration of 1 mM.

## Claims

1. A pharmaceutical composition containing an inhibitor of platelet aggregation, a fibrate and one or more pharmaceutically acceptable excipients.

2. The pharmaceutical composition according to claim 1, wherein the inhibitor of platelet aggregation is aspirin or clopidogrel.

3. The pharmaceutical composition according to claim 1 or 2, wherein the fibrate is fenofibrate or fenofibric acid.

4. The pharmaceutical composition according to claim 1 or 2, wherein the inhibitor of platelet aggregation is aspirin, the fibrate is fenofibrate, and the pharmaceutical composition contains no further pharmacologically active substances.

5. The pharmaceutical composition according to claim 1 or 2, wherein the inhibitor of platelet aggregation is clopidogrel, the fibrate is fenofibrate, and the pharmaceutical composition contains no further pharmacologically active substances.

6. Use of an inhibitor of platelet aggregation and a fibrate and one or more pharmaceutically acceptable excipients in the manufacture of a medicament for the prevention or treatment of cardiovascular diseases and events.

7. The use according to claim 6, wherein the inhibitor of platelet aggregation is aspirin or clopidogrel.

8. The use according to claim 6 or 7, wherein the fibrate is fenofibrate or fenofibric acid.

9. The use according to one of claims 6 to 8, wherein the inhibitor of platelet aggregation and the fibrate and are administered simultaneously or sequentially.
